(19) Europäisches Patentamt
European Patent Office
Office européen des brevets

(11) Publication number: **0 333 248 A2**

# (12) EUROPEAN PATENT APPLICATION

(21) Application number: 89200541.4

(22) Date of filing: 06.03.89

(51) Int. Cl.[4]: C11D 3/39 , C07C 143/38

(30) Priority: 17.03.88 GB 8806370
25.04.88 GB 8809729

(43) Date of publication of application:
20.09.89 Bulletin 89/38

(84) Designated Contracting States:
CH DE ES FR GB IT LI NL SE

(71) Applicant: UNILEVER NV
Burgemeester s'Jacobplein 1 P.O. Box 760
NL-3000 DK Rotterdam(NL)

(84) CH DE ES FR IT LI NL SE

Applicant: UNILEVER PLC
Unilever House Blackfriars P.O. Box 68
London EC4P 4BQ(GB)

(84) GB

(72) Inventor: Roberts, David William
26 Venables Drive Bebington
Wirral Merseyside L63 9LT(GB)
Inventor: Sims, Peter Stanford
71 Dee Banks
Chester Cheshire(GB)
Inventor: Thornthwaite, David William
Lorien Leighton Road
Neston Cheshire L64 3SF(GB)

(74) Representative: Tan, Bian An, Ir. et al
Unilever N.V. Patent Division P.O. Box 137
NL-3130 AC Vlaardingen(NL)

## (54) Bleach precursors and their use in bleaching and/or detergent composition.

(57) A novel peroxyacid bleach precursors compound is disclosed, having the structural formula :

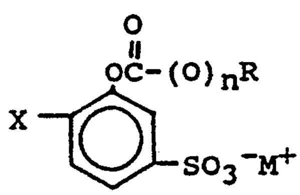

wherein n = 0 or 1; X is a methyl, ethyl, methoxy or ethoxy group; $M^+$ is hydrogen, alkali metal, earth alkali metal, ammonium or alkyl or hydroxyalkyl substituted ammonium cation; and R is an alkyl group containing 1 to 9 carbon atoms or a phenyl group. Bleaching-detergent compositions comprising a peroxide bleach compound and said peroxyacid bleach precursor are effective for use at the lower wash temperature region of from ambient to about 40 °C.

EP 0 333 248 A2

# Bleach precursors and their use in bleaching and/or detergent compositions

## Field of the Invention

This invention relates to novel peroxyacid bleach precursor compounds and to bleaching and/or detergent compositions employing these compounds in combination with a peroxide compound capable of yielding hydrogen peroxide in solution.

## Background to the Invention

It is well known that active oxygen-releasing peroxide compounds are effective bleaching agents. These compounds are frequently incorporated in detergent compositions for stain and soil removal. They have, however, an important limitation: the activity is extremely temperature-dependent. Thus, active oxygen-releasing bleaches are essentially only practical when the bleaching solution is heated above 60 °C. At a bleach solution temperature of about 60 °C, extremely high amounts of the active oxygen-releasing compounds must be added to achieve any bleaching effect. This is both economically and practically disadvantageous. As the bleach solution temperature is lowered below 60 °C, peroxide compounds, e.g. sodium perborate, are rendered ineffective, regardless of the level of peroxide compound added to the system. The temperature dependency of peroxide compounds is significant because such bleach compounds are commonly used as a detergent adjuvant in textile wash processes that utilize an automatic household washing machine operating at wash water temperatures of below 60 °C. Such wash temperatures are utilized because of textile care and energy considerations. Consequently, a constant need has developed of substances which render peroxide compound bleaches more effective at bleach solution temperatures below 60°. These substances are generally referred to in the art as bleach precursors, promoters or activators.

Typically, the precursor is a reactive compound of the N-acyl or O-acyl type that in alkaline solution containing a source of hydrogen peroxide, e.g. a persalt, such as sodium perborate, will generate the corresponding peroxyacid. The reaction involves nucleophilic substitution on to the precursor molecule by perhydroxide anions ($HOO^-$) and is facilitated by precursors having good leaving groups. Often this reaction is referred to as perhydrolysis. Numerous substances have been proposed in the art as effective bleach precursors, promoters or activators, such as disclosed in a series of articles by Allan H. Gilbert in Detergent Age, June 1967, pages 18-20, July 1967, pages 30-33, and August 1967, pages 26, 27 and 67; and further in GB patents 836,988; 864,798; 907,356; 1,003,310 and 1,519,351; German patent 3,337,921; EP-A-0,185,522; Ep-A-0,174,132; EP-A-0,120,591; and US patents 4,412,934 and 4,675,393.

Normally, the precursor is also a hydrolysable material which can react with moisture and alkaline components of the detergent composition during storage, forming non-reactive products. This reaction, referred to as hydrolysis, causes loss of precursor when incorporated in detergent compositions, the extent of which is highly dependent upon the ease at which the precusor undergoes the hydrolysis reaction.

Various means have been proposed in the art to protect the precursor from the aqueous and alkaline components of the detergent composition during storage. It should be appreciated, however, that the less stable to hydrolysis the precursor is, the more difficult it will be to achieve adequate protection.

It is believed that this may be one reason why only a few of the large number of proposed compounds have found commercial exploitation, of which N,N,N′,N′-tetraacetyl ethylene diamine (TAED), belonging to the type of N-acyl precursors, is the one most widely used in practice.

One drawback of TAED, however, is the sluggishness of the peroxyacid release from the reaction with the peroxide compound liberating hydrogen peroxide, such as sodium perborate, sodium percarbonate, sodium persilicate, urea peroxide and the like, resulting in a non-optimal bleaching effect. TAED can thus be classed as a slow-acting precursor.

Another drawback of TAED is that its solubility in water is rather poor, i.e. somewhere in the region of 1%, which is another reason for the non-optimal bleaching performance of TAED/hydrogen peroxide systems.

With the trend towards still lower fabric washing temperatures, to e.g. 40 °C and below, there is an incentive to improve on the bleaching performance of TAED/peroxide compound systems. One option is to replace TAED by a fast-acting precursor, i.e. one which releases peroxyacid faster than does TAED. Typical

fast-acting precursors are those O-acyl type compounds belonging to the class of acyloxy benzene sulphonates such as sodium n-nonanoyloxy benzene sulphonate, sodium 3,5,5-trimethyl hexanoyloxy benzene sulphonate, sodium acetoxy benzene sulphonate in which the acyloxy group is p-substituted.

These precursors are carboxylic esters as disclosed in e.g. GB Patent 864,798; US Patent 4,412,934; and EP-A 0,120,591.

A disadvantage of these esters, however, is that they tend to (per)-hydrolyse more readily than tetraacetyl ethylene diamine (TAED), and hence suffer from a more severe decomposition problem during storage.

Consequently, a constant need has developed of new and possibly better substances which render peroxide compound bleaches more effective at bleach solution temperatures in the region of from ambient to about 40° C.

It is an object of the present invention to provide novel fast-acting precursors of peroxyacids which are suitable for use in domestic laundry detergent compositions.

It is another object of the present invention to provide an effective bleaching and/or detergent composition comprising novel peroxyacid bleach precursors and having improved overall bleaching performance at the lower wash temperature region of from ambient to about 40° C.

By the term "fast-acting" is meant here that the precursor will have a rate of peroxyacid release which is at least 2 (two) times, preferably at least 5 (five) times, faster than TAED.

## Description of the Invention

It has been found that the above objects can be achieved if the peroxyacid bleach precursor is a compound having the following specific molecular structural formula :

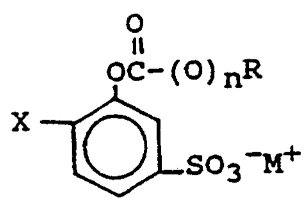

wherein n = 0 or 1; X is a methyl, ethyl, methoxy or ethoxy group; $M^+$ is hydrogen, alkali metal, earth alkali metal, ammonium or alkyl or hydroxyalkyl substituted ammonium cation, and R is an alkyl group containing 1 to 9 carbon atoms or a phenyl group.

Accordingly, the invention provides a novel peroxyacid bleach precursor compound having the structural formula:

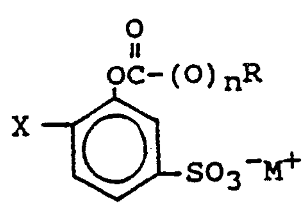

wherein n = 0 or 1; X is a methyl, ethyl, methoxy or ethoxy group; $M^+$ is hydrogen, alkali metal, earth alkali metal, ammonium or alkyl or hydroxyalkyl substituted ammonium cation; and R is an alkyl group containing 1 to 9 carbon atoms or a phenyl group.

Typical of the bleach precursors according to the invention is that, unlike the known acyloxy benzene sulphonates of the art, the ester and the sulphonate groups are 2,4-positioned (in meta-position) on the benzene nucleus, and that the benzene ring must contain a further substituent X in 1,2-position to the ester group.

It is this specific arrangement in the molecular structure that seems to make the compound surprisingly more robust against hydrolysis attack.

Preferred precursors are those of the above formula wherein X is a methyl or a methoxy group; and R

is a methyl, $C_8$-alkyl or phenyl group; and n = 0, with particular preference of R being methyl or phenyl.

The following compounds are illustrative of precursors within the present invention :

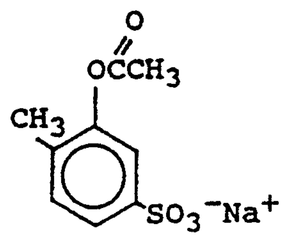

(I)

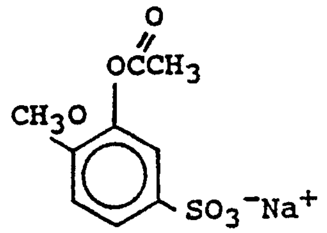

(II)

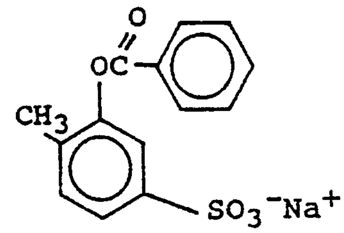

(III)

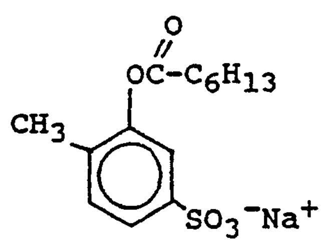

(IV)

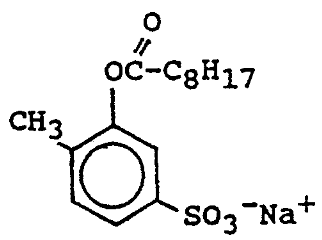

(V)

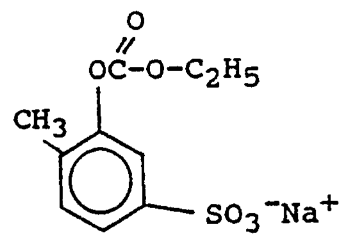

(VI)

These compounds of the invention are more soluble and much more reactive than TAED; they are relatively stable upon storage, both alone and when mixed with additional components in alkaline detergent compositions. The term stability, as used herein, means that the bleach precursor has a rate of hydrolysis lower than that of sodium-p-acetoxy benzene sulphonate.

Also systems comprising the peroxyacid bleach precursors and hydrogen peroxide liberating peroxide bleaching compounds show excellent bactericidal properties.

As explained above, the foregoing precursors may be incorporated in bleaching-detergent compositions which require as an essential component a peroxide bleaching compound capable of yielding hydrogen peroxide in an aqueous solution.

Hydrogen peroxide sources are well known in the art. They include the alkali metal peroxides, organic peroxide bleaching compounds such as urea peroxide, and inorganic persalt bleaching compounds, such as the alkali metal perborates, percarbonates, perphosphates and persulphates. Mixtures of two or more such compounds may also be suitable. Particularly preferred are sodium perborate tetrahydrate and, especially, sodium perborate monohydrate. Sodium perborate monohydrate is preferred because it has excellent storage stability while also dissolving very quickly in aqueous bleaching solutions. Rapid dissolution is believed to permit formation of higher levels of percarboxylic acid which would enhance surface bleaching performance.

Typically, the molar ratio of hydrogen peroxide (or a peroxide compound generating the equivalent amount of $H_2O_2$) to precursor will range from 0.5:1 to about 20:1, preferably 1:1 to 5:1, most preferably from 1:1 to 2:1.

A detergent formulation containing a bleach system consisting of an active oxygen-releasing material and a novel compound of the invention will usually also contain surface-active materials, detergency builders and other known ingredients of such formulations.

In such formulations the novel peroxyacid bleach precursor of the invention may be present at a level ranging from about 0.1% to 20% by weight, preferably from 0.5% to 10% by weight, particularly from 1% to 7.5% by weight, together with a peroxide bleaching compound, e.g. sodium perborate mono- or tetra-hydrate, the amount of which is usually within the range of from about 2% to 40%, preferably from about 4% to 30%, particularly from about 10% to 25% by weight.

The surface-active material may be naturally derived, such as soap, or a synthetic material selected from anionic, nonionic, amphoteric, zwitterionic, cationic actives and mixtures thereof. Many suitable actives are commercially available and are fully described in literature, for example in "Surface Active Agents and Detergents", Volumes I and II, by Schwartz, Perry and Berch. The total level of the surface-active material may range up to 50% by weight, preferably being from about 1% to 40% by weight of the composition, most preferably 4 to 25%.

Synthetic anionic surface-actives are usually water-soluble alkali metal salts of organic sulphates and sulphonates having alkyl radicals containing from about 8 to about 22 carbon atomm, the term alkyl being used to include the alkyl portion of higher aryl radicals.

Examples of suitable synthetic anionic detergent compounds are sodium and ammonium alkyl sulphates, especially those obtained by sulphating higher ($C_8$-$C_{18}$) alcohols produced, for example, from tallow or coconut oil; sodium and ammonium alkyl ($C_9$-$C_{20}$) benzene sulphonates, particularly sodium linear secondary alkyl ($C_{10}$-$C_{15}$) benzene sulphonates; sodium alkyl glyceryl ether sulphates, especially those esters of the higher alcohols derived from tallow or coconut oil and synthetic alcohols derived from petroleum; sodium coconut oil fatty acid monoglyceride sulphates and sulphonates; sodium and ammonium salts of sulphuric acid esters of higher ($C_9$-$C_{18}$) fatty alcohol alkylene oxide, particularly ethylene oxide, reaction products; the reaction products of fatty acids such as coconut fatty acids esterified with isethionic acid and neutralized with sodium hydroxide; sodium and ammonium salts of fatty acid amides of methyl taurine; alkane monosulphonates such as those derived by reacting alpha-olefins ($C_8$-$C_{20}$) with sodium bisulphite and those derived by reacting paraffins with $SO_2$ and $Cl_2$ and then hydrolyzing with a base to produce a random sulphonate; sodium and ammonium $C_7$-$C_{12}$ dialkyl sulfosuccinates; and olefin sulphonates, which term is used to describe the material made by reacting olefins, particularly $C_{10}$-$C_{20}$ alpha-olefins, with $SO_3$ and then neutralizing and hydrolyzing the reaction product. The preferred anionic detergent compounds are sodium ($C_{11}$-$C_{15}$) alkylbenzene sulphonates, sodium ($C_{16}$-$C_{18}$) alkyl sulphates and sodium ($C_{16}$-$C_{18}$) alkyl ether sulphates.

Examples of suitable nonionic surface-active compounds which may be used, preferably together with the anionic surface-active compounds, include in particular the reaction products of alkylene oxides, usually ethylene oxide, with alkyl ($C_6$-$C_{22}$) phenols, generally 5-25 EO, i.e. 5-25 units of ethylene oxides per molecule; the condensation products of aliphatic ($C_8$-$C_{18}$) primary or secondary linear or branched alcohols with ethylene oxide, generally 6-30 EO, and products made by condensation of ethylene oxide with the reaction products of propylene oxide and ethylene diamine. Other so-called nonionic surface-actives include alkyl polyglycosides, long chain tertiary amine oxides, long chain tertiary phosphine oxides and dialkyl sulphoxides.

Amounts of amphoteric or zwitterionic surface-active compounds can also be used in the compositions of the invention but this is not normally desired owing to their relatively high cost. If any amphoteric or zwitterionic detergent compounds are used, it is generally in small amounts in compositions based on the much more commonly used synthetic anionic and nonionic actives.

As stated above, soaps may also be incorporated in the compositions of the invention, preferably at a level of less than 25% by weight. They are particularly useful at low levels in binary (soap/anionic) or ternary mixtures together with nonionic or mixed synthetic anionic and nonionic compounds. Soaps which are used are preferably the sodium, or, less desirably, potassium salts of saturated or unsaturated $C_{10}$-$C_{24}$ fatty acids or mixtures thereof. The amount of such soaps can be varied between about 0.5% and about 25% by weight, with lower amounts of about 0.5% to about 5% being generally sufficient for lather control. Amounts of soap between about 2% and about 20%, especially between about 5% and about 10%, are used to give a beneficial effect on detergency. This is particularly valuable in compositions used in hard water when the soap acts as a supplementary builder.

The detergent compositions of the invention will normally also contain a detergency builder. Builder materials may be selected from 1) calcium sequestrant materials, 2) precipitating materials, 3) calcium ion-exchange materials and 4) mixtures thereof.

Examples of calcium sequestrant builder materials include alkali metal polyphosphates, such as sodium tripolyphosphate; nitrilotriacetic acid and its water-soluble salts; the akali metal salts of carboxymethyloxy succinic acid, ethylene diamine tetraacetic acid, oxydisuccinic acid, mellitic acid, benzene polycarboxylic acids, citric acid; and polyacetal carboxylates as disclosed in US patents 4,144,226 and 4,146,495.

Examples of precipitating builder materials include sodium orthophosphate, sodium carbonate and long chain fatty acid soaps.

Examples of calcium ion-exchange builder materials include the various types of water-insoluble crystalline or amorphous aluminosilicates, of which zeolites are the best known representatives.

In particular, the compositions of the invention may contain any one of the organic or inorganic builder materials, such as sodium or potassium tripolyphosphate, sodium or potassium pyrophosphate, sodium or potassium orthophosphate, sodium carbonate, the sodium salt of nitrilotriacetic acid, sodium citrate, carboxymethyl malonate, carboxymethyloxy succinate and the water-insoluble crystalline or amorphous aluminosilicate builder materials, or mixtures thereof.

These builder materials may be present at a level of, for example, from 5 to 80% by weight, preferably from 10 to 60% by weight.

Apart from the components already mentioned, the detergent compositions of the invention can contain any of the conventional additives in the amounts in which such materials are normally employed in fabric washing detergent compositions. Examples of these additives include lather boosters, such as alkanolamides, particularly the monoethanol amides derived from palmkernel fatty acids and coconut fatty acids, lather depressants, such as alkyl phosphates and silicones, anti-redeposition agents, such as sodium carboxymethyl cellulose and alkyl or substituted alkyl cellulose ethers, other stabilizers, such as ethylene diamine tetraacetic acid, fabric softening agents, inorganic salts, such as sodium sulphate, and, usually present in very small amounts, fluorescent agents, perfumes, enzymes, such as proteases, cellulases, lipases and amylases, germicides and colourants.

The peroxyacid bleach precursors described herein are useful in a variety of cleaning products. These include laundry detergents, laundry bleaches, hard surface cleaners, toilet bowl cleaners, automatic dishwashing compositions and even denture cleaners. Precursors of the present invention can be introduced in a variety of product forms including powders, on sheets or other substrates, in pouches, in tablets or in non-aqueous liquids, such as liquid nonionic detergents.

Generally, for reasons of stability and handling, the bleach precursors will advantageously be presented in the form of particulate bodies comprising said bleach precursor and a binder or agglomerating agent. Many and diverse methods of preparing such precursor particulates have been described in various patent literature documents, such as e.g. in Canadian Patent N° 1,102,966; GB Patent N° 1,561,333; US Patent N° 4,087,369; EP-A-0,240,057; EP-A-0,241,962; EP-A-0,101,634 and EP-A-0,062,523. Each of these methods may be selected and applied to the bleach precursor of the invention.

Particulates incorporating the precursors of the present invention are normally added to the spray-dried portion of the detergent composition with the other dry-mix ingredients, such as enzymes, inorganic peroxygen bleaches and suds depressants. It will be appreciated, however, that the detergent composition to which the precursor particulates are added may itself be made in a variety of ways, such as dry-mixing, agglomeration extrusion, flaking etc., such ways being well known to those skilled in the art and not forming part of the present invention.

The peroxyacid precursors of the present invention can also be incorporated in detergent additive products. Such additive products are intended to supplement or boost the performance of conventional detergent compositions and may contain any of the components of such compositions, although they will not comprise all of the components present in a fully formulated detergent composition. Additive products in accordance with this aspect of the invention will normally be added to an aqueous liquor containing a source of (alkaline) hydrogen peroxide, although in certain circumstances a source of alkaline hydrogen peroxide may be included in the product.

Additive products in accordance with this aspect of the invention may comprise the compound alone in combination with a carrier, such as a compatible particulate substrate, a flexible non-particulate substrate or a container (e.g. pouch or sachet).

Examples of compatible particulate substrates include inert materials, such as clays and other aluminosilicates including zeolites both natural and synthetic of origin. Other compatible particulate carrier materials include hydratable inorganic salts, such as phosphates, carbonates and sulphates.

Additive products enclosed in bags or containers can be manufactured such that the containers prevent egress of their contents when dry but are adapted to release their contents on immersion in an aqueous solution.

In a further specific embodiment, the peroxyacid precursors of the invention are particularly suitable for incorporation in so-called non-aqueous liquid laundry detergent compositions together with a peroxide bleaching compound, e.g. sodium perborate, to impart an effective cleaning and stain-removing capacity to the products on fabrics and textiles.

Non-aqueous liquid detergent compositions including paste-like and gelatinous detergent compositions in which the precursor compounds can be incorporated are known from the art and various formulations have been proposed, e.g. in US Patents 2,864,770; 2,940,938; 4,772,412; 3,368,977; GB-A-1,205,711; 1,270,040; 1,292,352; 1,370,377; 2,194,536; DE-A-2,233,771; and EP-A-0,028,849.

These are compositions which normally comprise a non-aqueous liquid medium with or without a solid phase dispersed therein. The non-aqueous liquid medium may be a liquid surfactant, preferably a liquid nonionic surfactant; a non-polar liquid medium, e.g. liquid paraffin; a polar solvent, e.g. polyols, such as glycerol, sorbitol, ethylene glycol, optionally combined with low-molecular monohydric alcohols, e.g. ethanol or isopropanol; or mixtures thereof.

The solid phase can be builders, alkalis, abrasives, polymers, clays, other solid ionic surfactants, bleaches, enzymes, fluorescent agents and other usual solid detergent ingredients.

The precursor compounds of the invention may be prepared and synthesized by way of the following

example of a chemical reaction scheme :

Preparation of compound (III)

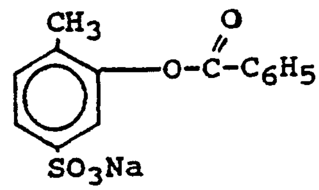

Sodium-1-methyl-2-benzoyloxybenzene-4-sulphonate

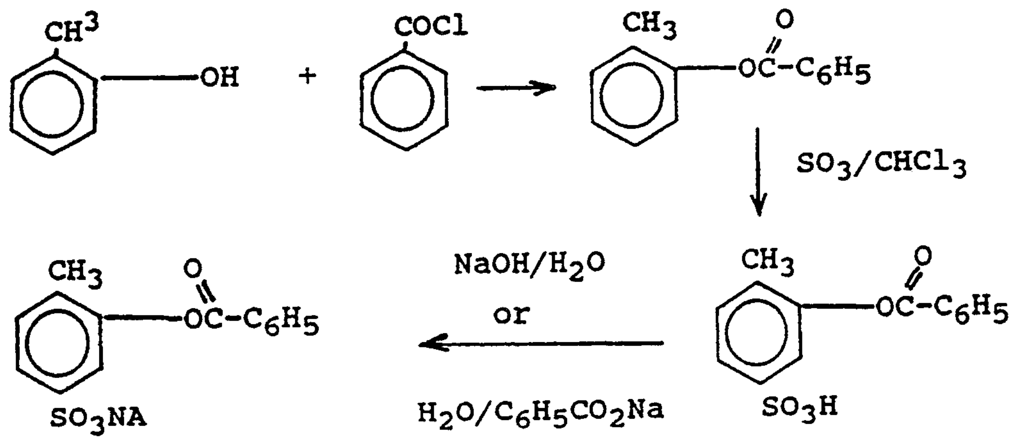


Description of process :

2-Methyl phenol (o-cresol) (38g; 0.35M) was added to benzoyl chloride (50 g; 0.355M), and this mixture was heated on a water bath (50 ° C) for a period of 6 h. After cooling, diethyl ether (200 ml) was added and this solution was washed with sodium carbonate (200 ml; 40 g in water), water (200 ml) and then dried over magnesium sulphate. The solution was filtered and concentrated under reduced pressure to yield an oil (74 g, 99%) IR 1740 $cm^{-1}$ This oily ester was dissolved in anhydrous chloroform (100 ml) and maintained at 40 ° C while liquid sulphur trioxide (154 ml (30.8 g); 0.385M) was added dropwise with stirring over a period of 1 h. The mixture was stirred for a further hour at 40 ° C, after which it was poured into ice water (200 ml) containing sodium benzoate (55.4 g; 0.385M) with stirring for 30 min. The mixture was separated and the chloroform layer discarded. The aqueous phase was washed with ether (2 x 200 ml), then freeze-dried to yield a white solid (112 g) IR 1735, 1200, 1030 $cm^{-1}$. 'HNMR ($D_2O$) consistent with structure.

## EXAMPLE I

The hydrolysis of various bleach precursors was measured by using the following technique.

1 gram of sodium lauryl sulphate and 2 grams of sodium metaborate tetrahydrate were dissolved in 1000 ml of double-distilled deionised water; this solution was used in the reference cell of the spectrophotometer. To 800 ml of the stirred solution was added sufficient precursor to give an optical density of 0.4 to 0.8, and the solution was passed through a flow-cell in the spectrophotometer. The decomposition (hydrolysis) of the precursor was monitored by measuring the decrease in optical density at the wavelength of maximum absorbance.

The following bleach precursors were used:

1) MOABS = sodium-1-methoxy-2-acetoxybenzene-4-sulphonate
2) MABS = sodium-1-methyl-2-acetoxybenzene-4-sulphonate

3) SABS = sodium-1-acetoxybenzene-4-sulphonate
4) MBBS = sodium-1-methyl-2-benzoyloxybenzene-4-sulphonate
5) BOBS = sodium-1-benzoyloxy-4-sulphonate.

The SABS is the control for MOABS and MABS, which are precursors of peracetic acid, and BOBS is the control for MBBS, which is a perbenzoic acid precursor.

The results are as tabulated below:

Table I

| Precursor | Rate (/min.) | loss after 60 min. (%) |
|---|---|---|
| 1) MOABS | 0.0083 | 36 |
| 2) MABS | 0.0037 | 19 |
| 3) SABS | 0.0154 | 57 |
| 4) MBBS | 0.0027 | 13 |
| 5) BOBS | 0.0038 | 20 |

These results confirm that the bleach precursors 1), 2) and 4) of the invention are more stable to hydrolysis than SABS, and that each precursor of the same peracid is more hydrolytically stable than its control-precursor of the art.

## EXAMPLE II

The following granular detergent composition was prepared by spray-drying an aqueous slurry:

| Composition | Parts by weight |
|---|---|
| sodium alkyl benzene sulphonate | 6.0 |
| $C_{14-15}$ alcohol / 7 ethylene oxide | 7.0 |
| sodium soap | 1.6 |
| zeolite | 24.0 |
| alkaline silicate | 0.5 |
| polyacrylate | 4.0 |
| sodium carbonate | 8.0 |
| sodium carboxymethylcellulose | 0.5 |
| ethylene diamine tetraacetate | 0.2 |
| fluorescer | 0.2 |
| salts | 0.7 |

To this base powder were added 15 parts of sodium perborate monohydrate, and an amount of precursor at a molar ratio of precursor to perborate of 1:9.

Bleaching tests were carried out with the final powder formulation using different precursors, in a Tergotometer heat-up wash to 40°C in 24°FH water at a dosage of 5 g/l. Tea-stained test pieces were used as the bleach monitor. The bleaching efficiencies were determined using an Elrepho reflectometer and the results expressed as ΔR 460* are shown in the following Table II.

Table II

| Precursor | ΔR 460* |
|---|---|
| 1) MOABS | 3.5 |
| 2) MABS | 3.0 |
| 3) SABS | 3.7 |
| 4) MBBS | 4.5 |
| 5) BOBS | 4.6 |
| 6) TAED | 2.0 |

These results show that there is no significant difference in performance between any of the precursors of the invention and the relevant fast-acting precursor control, i.e. 1) and 2) vs. 3); and 4) vs. 5). The precursors 1) to 5) were all superior to TAED at 40 °C.

## Claims

1. A peroxyacid bleach precursor compound having the structural formula :

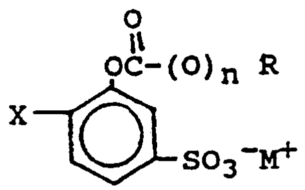

wherein n = 0 or 1; X is a methyl, ethyl, methoxy or ethoxy group; $M^+$ is hydrogen, alkali metal, earth alkali metal, ammonium or alkyl or hydroxyalkyl substituted ammonium cation; and R is an alkyl group containing 1 to 9 carbon atoms or a phenyl group.

2. A bleach precursor according to Claim 1, characterized in that X is a methyl or methoxy group; R is a methyl, $C_8$-alkyl or phenyl group; and n = 0.

3. A bleaching-detergent composition comprising a peroxide bleaching compound capable of yielding hydrogen peroxide in aqueous solution and a peroxyacid bleach precursor in a molar ratio ranging from 0.5:1 to about 20:1, characterized in that said peroxyacid bleach precursor is a compound having the formula :

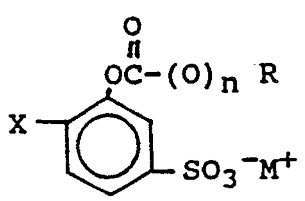

wherein n = 0 or 1; X is a methyl, ethyl, methoxy or ethoxy group; $M^+$ is hydrogen, alkali metal, earth alkali metal, ammonium or alkyl or hydroxyalkyl substituted ammonium cation; and R is an alkyl group containing 1 to 9 carbon atoms or a phenyl group.

4. A bleaching-detergent composition according to Claim 3, characterized in that X is a methyl or methoxy group; R is a methyl, $C_8$-alkyl or phenyl group; and n = 0.

5. A bleaching-detergent composition according to Claim 3 or 4, characterized in that it further contains a surface-active material and a detergency builder.

6. A bleaching-detergent composition according to Claim 3, 4 or 5, characterized in that the peroxide bleaching compound is sodium perborate monohydrate.